# EUROPEAN PATENT SPECIFICATION

(11) **EP 4 513 163 B1**
(45) Date of publication and mention of the grant of the patent: **01.04.2026**
(21) Application number: 23382548.8
(22) Date of filing: 07.06.2023
(51) Int. Cl.: G01N 1/40, G01N 35/00, G01N 33/15

(54) **A FILTER CARTRIDGE ASSEMBLY AND METHOD OF OBTAINING SAID FILTER CARTRIDGE ASSEMBLY**
FILTERPATRONENANORDNUNG UND VERFAHREN ZUR HERSTELLUNG DER FILTERPATRONENANORDNUNG
ENSEMBLE CARTOUCHE FILTRANTE ET PROCÉDÉ D'OBTENTION DUDIT ENSEMBLE CARTOUCHE FILTRANTE

(43) Date of publication of application: 26.02.2025
(73) Proprietor: Giménez Guasch, Francesc Xavier, 08330 Premià de Mar (ES); Carabaza Bravo, Maria Assumpta, 08330 Premià de Mar (ES)
(72) Inventor: Giménez Guasch, Francesc Xavier, 08330 Premià de Mar (ES); Carabaza Bravo, Maria Assumpta, 08330 Premià de Mar (ES)
(74) Representative: Ponti & Partners, S.L.P

(56) References cited:
- US-A1- 2003 064 423
- US-A1- 2010 080 733
- US-A1- 2021 053 048

## Description

### Field of the Invention

The present invention generally relates, in a first aspect, to a filter cartridge assembly for automated sample preparation in dissolution testing.

In a second aspect, the present invention relates to a method of obtaining the filter cartridge assembly of the first aspect.

### Background of the Invention

Automated sample stations are known that enable automated sample preparation in dissolution testing. Samples are filtered, withdrawn, and archived for future HPLC and UV analysis. The company Agilent technologies Inc. commercializes one of those automated sample stations known as 850-DS Dissolution sampling station.

Filter cartridges assemblies have been provided for using on filter modules of the above-mentioned automated sample stations or similar stations. Each filtration assembly contains a plurality of filter cartridges consisting of modified syringe filters which are incorporated on a flat plate.

The compact arrangement of the existent filter cartridge assemblies makes it much easier for the automated equipment to process and filter samples over traditional automation-ready filters.

However, the existent filter assemblies have the drawback that each filter cartridge is manufactured from a known syringe filter which needs to be modified by cutting or removing its Luer fittings before being incorporated on the flat plate so as to keep the filtration assembly flat for said filtration assembly to be able to be inserted in a filter module of the automated sample station. Besides, the existent filtration assemblies have the drawback that they are not suitable for filtering at high pressure operation condition. In addition, it has been found that the operating pressure of filter cartridges of the same filter assembly is not uniform.

It is necessary to offer an alternative to the state of the art, which covers the gaps mentioned above, particularly by providing a compact filter cartridge assembly which does not have the above-mentioned drawbacks and limitations of the existent filtration plates.

US 2021/053048 A1 discloses a filter cartridge assembly according to the preamble of claim 1.

### Description of the Invention

To that end, the present invention relates, in a first aspect, to a filter cartridge assembly for automated sample preparation in dissolution testing according to claim 1 and comprising a plurality of filter cartridges, each of said filter cartridges including a liquid path for a liquid sample to be filtrated through a filter medium, wherein the filter cartridge assembly comprises a one-piece base part defining a plurality of preformed filter housings, wherein each of said preformed filter housings forms an integral part of one of said filter cartridges of said plurality of filter cartridges.

In contrast to the filtration assemblies known in the prior art, the filter cartridge assembly of the first aspect of the present invention comprises
□ a filter seal to seal respective filter caps to each of said preformed filter housings, wherein each of said preformed filter housings comprises a sealing seat where the filter seal is bonded to the one-piece base part to seal each filter cap to each of those preformed filter housings,
□ wherein the liquid path of each filter cartridge comprises a filter inlet arranged on the filter cap, and a filter outlet arranged on the respective preformed filter housing, wherein said filter inlet is configured for receiving liquid from a syringe injection device of an automated dissolution sample station, and
□ wherein said filter cartridge assembly is configured for being able to be inserted into a filter module of the automated dissolution sample station.

Thanks to the claimed features, the present invention provides a filter cartridge assembly with an improved sealing capacity. Besides, the filter cartridge assembly is suitable for using at high pressure operation condition since the particular design enables the filter assembly to sustain to high pressure, for example, to a high pressure of 7 or 10 bar at 23°C. Furthermore, it has been shown that the operating pressure of all filter cartridges of the same filter assembly remains homogeneous.

For an embodiment of the filter cartridge assembly, each filter cap includes a peripheral tab configured and arranged so that, when assembled to its corresponding preformed filter housing, the peripheral tab rests on a portion of the sealing seat and is bonded to the filter seal.

Thus, the filter seal keeps bonded to both the one-piece base part and to the filter cap to efficiently and robustly sealing each filter cartridge.

Advantageously, each of said preformed filter housings further comprises a filter seat for the filter medium, and a peripheral protruding portion of each filter cap clamps said filter medium in between the filter seat of each preformed filter housing and the peripheral protruding portion of each filter cap.

For an embodiment, the one-piece base part comprises a plurality of interconnecting sealing seat portions where the filter seal is bonded to the one-piece base part. Those interconnecting sealing seat portions are arranged between at least two preformed filter housings and a portion of the filter seal is bonded to those interconnecting sealing seat portions to reinforce the sealing of the filter cartridge assembly.

Preferably, the filter seal is a one-piece filter seal and, according to an embodiment, the filter seal is bonded to the one-piece base part by overmolding.

For a preferred embodiment, the filter seal has a color, preferably, a color different from the color of each filter cap and the color of the one-piece base part. This color, or different color, may be used as a code to help identifying the type of filter medium each filter cartridge contains since this filter medium may vary on composition, filter diameter and pore size according to the method of dissolution sampling to be processed.

Preferably, the one-piece base part comprises at least one reinforced rib arranged between said preformed filter housings. Advantageously, the outer lower surface of the one-piece base part comprises a plurality of reinforced ribs arranged in between said preformed filter housings.

As stated above, the liquid path of each filter cartridge comprises a filter inlet arranged on the filter cap and a filter outlet arranged on the respective preformed filter housing, wherein said filter inlet is configured for receiving liquid from a syringe injection device of an automated dissolution sample station. Advantageously, the filter inlet comprises a stop for the injection tube of the syringe injection device to prevent the injection tube from accidentally contacting the filter medium.

For an embodiment, each preformed filter housing comprises a channeled surface defining a channeled flow path for guiding the filtered sample to the filter outlet.

Preferably, the filter medium comprises a filter membrane configured and arranged on each of said preformed filter housings for the removal of particulate impurities and/or bacteria from liquid samples to be filtered.

The most common used materials for the filter membrane are; Cellulose acetate, Cellulose nitrate, Glass fiber, Mixed cellulose esters (MCE), Nylon, PES, Polypropylene, Polysulfone, PTFE - Hydrophilic, PTFE - Hydrophobic, PTFE, all (Hydrophobic and Hydrophilic), PVDF and Regenerated Cellulose.

As regards pore size, 0.45 µm and 0.2/0.22 µm are the two most frequently used membrane pore sizes for research and medical applications. 0.45 µm membranes are typically used for general filtration and particle removal applications while 0.2/0.22 µm membranes, or sterilizing-grade membranes, are most used for solution sterilization (bacteria removal).

According to one embodiment, the one-piece base part of the filter cartridge assembly of the present invention is made of polymeric material selected among at least one of polypropylene, polyethylene, and acrylic, and, advantageously, the filter seal of the filter cartridge assembly is made of polymeric material selected among at least one of polypropylene, polyethylene and acrylic.

The present invention also relates, in a second aspect, to a method of obtaining a filter cartridge assembly for automated sample preparation in dissolution testing according to claim 11, wherein the assembly includes a plurality of filtered cartridges and each of said filter cartridges includes a liquid path for a liquid sample to be filtrated through a filter medium, the method comprising the steps of;
a. providing a one-piece base part defining a plurality of preformed filter housings, wherein each preformed filter housing forms an integral part of a filter cartridge of said plurality of filter cartridges,
b. arranging individual filter mediums on each of said plurality of preformed filter housings,
c. covering each of said plurality of preformed filter housings with a respective filter cap, and
d. sealing the respective filter caps to each of said preformed filter housings by bonding a filter seal, preferably, one-piece filter seal, to respective sealing seats of said preformed filter housings.

The claimed method allows to obtain a filter cartridge assembly wherein the filter caps of the filter cartridges are sealed by bonding the filter seal, preferably, a one-piece filter seal, to sealing seats arranged on the one-piece base part. Thanks to these features, a filter cartridge assembly is obtained with an improved sealing capacity. Besides, the filter assembly is more robust than the existent known assemblies and enables to sustain to high pressure. Furthermore, as previously stated, it has been shown that the operating pressure of all the filter cartridges of the same filter assembly remains homogeneous.

Preferably, step d) comprises bonding by overmolding the filter seal, preferably, one-piece filter seal, to the respective sealing seats of said preformed filter housings.

For an embodiment, the method of the present invention comprises the step of prestressing the one-piece base part before bonding, preferably, by heating and overmolding, the filter seal to the respective sealing seats of said preformed filter housings.

### Brief Description of the Drawings

The previous and other advantages and features will be better understood from the following detailed description of embodiments, with reference to the attached drawings, which must be considered in an illustrative and non-limiting manner, in which:
Figure 1 is a schematic exploded perspective view of an embodiment of the filter cartridge assembly of the present invention that shows the one-piece base part defining the plurality of preformed filter housings and the one-piece filter seal to be bonded to the one-piece base part. For the sake of clarity only two filter medium and two respective filter caps have been represented. The filter caps of this embodiment are made of a material that allows the filter membrane to be seen from the outside of the filter assembly.
Figure 2 is a plan view of the filter cartridge assembly of Figure 1 including eight filter cartridges.
Figure 3 is a perspective upper view of the filter cartridge assembly of Figure 1, including eight filter cartridges.
Figure 4 is a perspective lower view of the filter cartridge assembly of Figure 1 that shows the outer lower surface of the one-piece base part defining the cavities of the eight preformed filter housings.
Figure 5 is a detailed view showing a cross-section of one of the filter cartridges of the filter cartridge assembly of Figure 1.

### Detailed Description of Several Embodiments

For the embodiment illustrated in the appended Figures, the filter cartridge assembly 1 includes a plurality of filter cartridges 2 and each of said filter cartridges 2 includes; a filter medium configured by way of a filter membrane 3 arranged in between a filter cap 4 and a preformed filter housing 5, and a liquid path 6 for a liquid sample to be filtrated through the filter membrane 3.

The filter cartridge assembly 1 is configured for being able to be inserted into a filter module (not shown) of an automated dissolution sample station (not shown) to carry out filtration of a sample to remove particulate impurities.

The liquid path 6 of each filter cartridge 2 comprises a filter inlet 6a arranged on the filter cap 4 and a filter outlet 6b arranged on the respective preformed filter housing 5. For the illustrated embodiment, the filter inlet 6a is configured for receiving liquid from a syringe injection device (not shown) of an automated dissolution sample station (not shown).

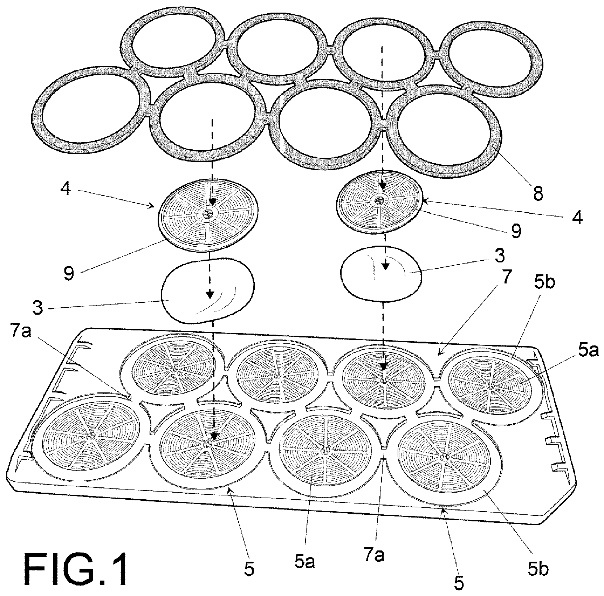

As shown in Figure 1, a one-piece base part 7 defines the plurality of preformed filter housings 5. Each preformed filter housing 5 comprises a channeled surface 5a defining a channeled flow path for guiding the filtered sample to the filter outlet 6b. Besides, each preformed filter housing 5 includes a sealing seat 5b where a filter seal 8 is bonded to the one-piece base part 7 to seal the corresponding filter cap 4 to the corresponding preformed filter housing 5.

For the illustrated embodiment, the filter seal 8 is a one-piece filter seal and the one-piece base part 7 comprises a plurality of interconnecting sealing seat portions 7a where the filter seal 8 is bonded to the one-piece base part 7. Those interconnecting sealing seat portions 7a are arranged between preformed filter housings 5 so that a portion of the filter seal 8 is bonded to those interconnecting sealing seat portions 7a to reinforce the sealing of all filter cartridges 2 of the filter cartridge assembly 1.

For a preferred embodiment, the one-piece filter seal 8 is bonded to the one-piece base part 7 by overmolding. However, other ways of bonding the filter seal 8 to the one-piece base part 7 may be envisaged.

In the illustrated figures, the one-piece filter seal 8 comprises a material that has a color, preferably, a color different from the color of each filter cap 4 and the color of the one-piece base part 7. As previously stated, the different color may be used as a code to help identifying the type of filter membrane 3 each filter cartridge 2 contains since the filter membrane 3 may vary on composition, filter diameter and pore size according to the method of dissolution sampling to be processed.

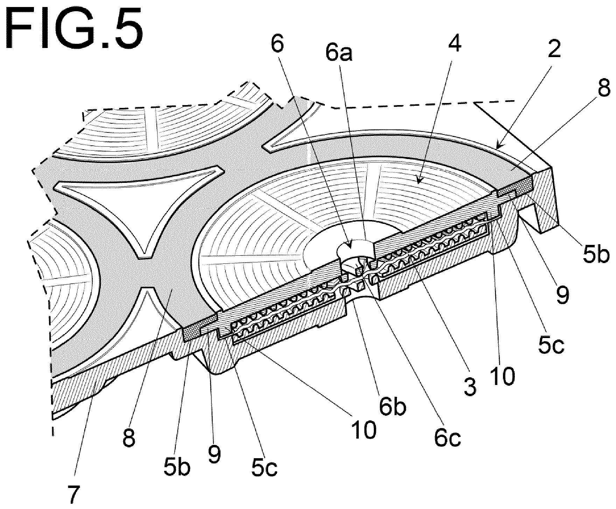

As shown in Figures 1 and 5, each filter cap 4 includes a peripheral tab 9 configured and arranged so that, when assembled to its corresponding preformed filter housing 5, the peripheral tab 9 rests on a portion of the sealing seat 5b and is bonded to the filter seal 8. Thanks to these features, the filter seal 8 keeps bonded to both the one-piece base part 7 and to the filter cap 4 to efficiently and robustly sealing each filter cartridge 2.

As shown in detail in Figure 5, each preformed filter housing 5 further comprises a filter seat 5c for the filter membrane 3, and each filter cap 4 comprises a protruding peripheral portion 10 configured to clamp the filter membrane 3 in between the filter seat 5c of each preformed filter housing 5 and the peripheral protruding portion 10 of the filter cap 4. This Figure 5 also shows the filter inlet 6a and filter outlet 6b of the filter cartridge 2 which are arranged aligned. The filter inlet 6a includes a stop member 6c for the injection tube of the syringe injection device (not shown) to prevent the injection tube from accidentally contacting the filter membrane 3.

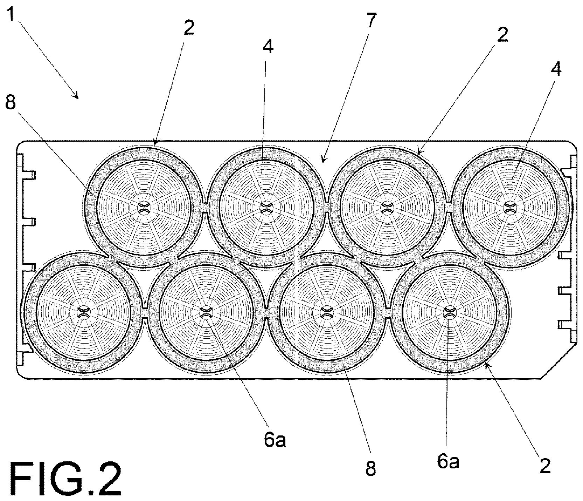

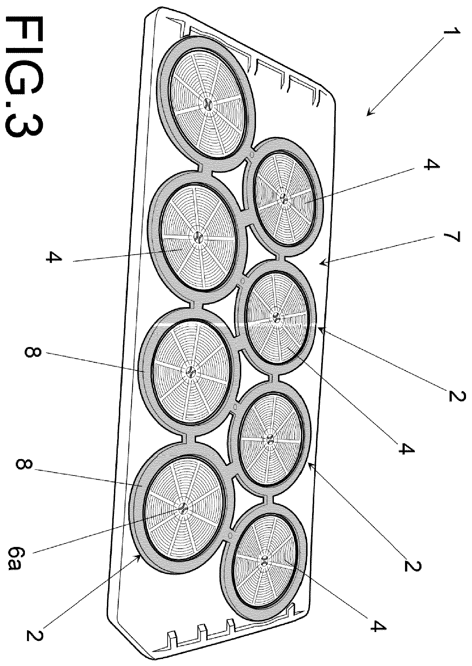

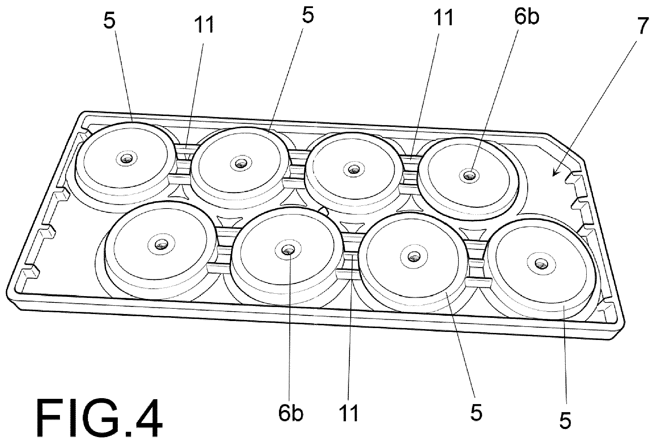

Figure 4 is a perspective lower view of the filter cartridge assembly 1 of Figures 2 and 3 that shows the outer lower surface of the one-piece base part 7 defining cavities corresponding to eight preformed filter housings 5. It also shows a plurality of reinforced ribs 11 arranged between the preformed filter housings 5 in order to reinforce the structure of the one-piece base part 7 while keeping the material required to manufacture the one-piece base part 7 to a minimum.

As previously disclosed, the present invention provides, in a second aspect, a method of obtaining a filter cartridge assembly 1 for automated sample preparation in dissolution testing, wherein the assembly 1 includes a plurality of filtered cartridges 2 and each of said filter cartridges 2 includes a liquid path 6 for a liquid sample to be filtrated through a filter membrane 3, comprising the steps of;
a. providing a one-piece base part 7 defining a plurality of preformed filter housings 5, wherein each preformed filter housing 5 forms an integral part of a filter cartridge 2,
b. arranging individual filter membranes 3 on each of said plurality of preformed filter housings 5,
c. covering each of said plurality of preformed filter housings 5 with a respective filter cap 4, and
d. sealing the respective filter caps 4 to each of said preformed filter housings 5 by bonding a filter seal 8, preferably, one-piece filter seal, to respective sealing seats 5b of said preformed filter housings 5.

For an embodiment, the method also comprises the step of prestressing the one-piece base part 7 before bonding, preferably, by heating and overmolding, the filter seal 8 to the respective sealing seats 5b of the preformed filter housings 5 defined by the one-piece base part 7.

Thanks to these features, a filter cartridge assembly 1 is obtained with an improved sealing capacity. Besides, the filter cartridge assembly 1 is more robust than the existent known assemblies and enables to sustain to high pressure. Furthermore, as previously stated, it has been shown that the operating pressure between the filter cartridges 2 of the same filter assembly 1 remains homogeneous.

A person skilled in the art could introduce changes and modifications in the embodiments described without departing from the scope of the invention as it is defined in the attached claims.

## Claims

1. A filter cartridge assembly (1) for automated sample preparation in dissolution testing comprising a plurality of filter cartridges (2), each of said filter cartridges (2) including a liquid path (6) for a liquid sample to be filtrated through a filter medium (3), wherein the filter cartridge assembly (1) comprises
□ a one-piece base part (7) defining a plurality of preformed filter housings (5), wherein each of said preformed filter housings (5) forms an integral part of one of said filter cartridges (2) of said plurality of filter cartridges (2), and
**characterized in that** the filter cartridge assembly (1) further comprises
□ a filter seal (8) to seal respective filter caps (4) to each of said preformed filter housings (5), wherein each of said preformed filter housings (5) comprises a sealing seat (5b) where the filter seal (8) is bonded to the one-piece base part (7) to seal each filter cap (4) to each of said preformed filter housings (5),
□ wherein the liquid path (6) of each filter cartridge (2) comprises a filter inlet (6a) arranged on the filter cap (4), and a filter outlet (6b) arranged on the respective preformed filter housing (5), wherein said filter inlet (6a) is configured for receiving liquid from a syringe injection device of an automated dissolution sample station, and
□ wherein said filter cartridge assembly (1) is configured for being able to be inserted into a filter module of the automated dissolution sample station.

2. A filter cartridge assembly (1) according to claim 1, wherein each filter cap (4) includes a peripheral tab (9) configured and arranged so that, when assembled to its corresponding preformed filter housing (5), the peripheral tab (9) rests on a portion of the sealing seat (5b) and is bonded to the filter seal (8).

3. A filter cartridge assembly (1) according to any of claims 1 to 2, wherein each of said preformed filter housings (5) further comprises a filter seat (5c) for the filter medium (3), and wherein a peripheral protruding portion (10) of each filter cap (4) clamps said filter medium (3) in between the filter seat (5c) of each preformed filter housing (5) and the peripheral protruding portion (10) of each filter cap (4).

4. A filter cartridge assembly (1) according to any of claims 1 to 3, wherein said one-piece base part (7) comprises a plurality of interconnecting seat portions (7a) where the filter seal (8) is also bonded to the one-piece base part (7).

5. A filter cartridge assembly (1) according to any of claims 1 to 4, wherein the filter seal is a one-piece filter seal (8) for sealing the filter caps (4) of the plurality of filter cartridges (2).

6. A filter cartridge assembly (1) according to any of claims 1 to 5, wherein the filter seal (8) is bonded by overmolding to the one-piece base part (7).

7. A filter cartridge assembly (1) according to any of claims 1 to 6, wherein the filter seal (8) has a color, preferably, a color different from the color of each filter cap (4) and the color of the one-piece base part (7).

8. A filter cartridge assembly (1) according to any of claims 1 to 7, wherein the one-piece base part (7) comprises at least one reinforced rib (11) arranged between said preformed filter housings (5).

9. A filter cartridge assembly (1) according to any of claims 1 to 8, wherein each preformed filter housing (5) comprises a channeled surface (5) defining a channeled flow path for guiding the filtered sample to the filter outlet (6b).

10. A filter cartridge assembly (1) according to any of claims 1 to 9, wherein the filter medium comprises a filter membrane (3) configured and arranged on each of said preformed filter housings (5) for the removal of particulate impurities and/or bacteria from liquid samples to be filtered.

11. A method of obtaining a filter cartridge assembly (1) for automated sample preparation in dissolution testing according to any of claims 1 to 10, wherein said assembly includes a plurality of filtered cartridges (2) and each of said filter cartridges (2) includes a liquid path (6) for a liquid sample to be filtrated through a filter medium (3), comprising the steps of;
a. providing a one-piece base part (7) defining a plurality of preformed filter housings (5), wherein each preformed filter housing (5) forms an integral part of one filter cartridge (2) of said plurality of filter cartridges (2),
b. arranging individual filter mediums (3) on each of said plurality of preformed filter housings (5),
c. covering each of said plurality of preformed filter housings (5) with a respective filter cap (4), and
d. sealing the respective filter caps (4) to each of said preformed filter housings (5) by bonding a filter seal (8) to respective sealing seats (5b) of said preformed filter housings (5).

12. A method according to claim 11, wherein step d) comprises bonding by overmolding the filter seal (8) to the respective sealing seats (5b) of said preformed filter housings (5).

13. A method according to any of claims 11 or 12, comprising the step of prestressing the one-piece base part (7) before bonding the filter seal (8) to the respective sealing seats (5b) of said preformed filter housings (5).

## Patentansprüche

1. Filterpatronenanordnung (1) für automatisierte Probenpräparation bei der Auflösungsprüfung, die mehrere Filterpatronen (2) umfasst, wobei jede der Filterpatronen (2) einen Flüssigkeitspfad (6) für eine zu filternde Flüssigkeitsprobe durch ein Filtermedium (3) enthält, wobei die Filterpatronenanordnung (1) umfasst:
- ein einteiliges Basisteil (7), das mehrere vorgeformte Filtergehäuse (5) definiert, wobei jedes der vorgeformten Filtergehäuse (5) einen integralen Teil einer der Filterpatronen (2) der mehreren Filterpatronen (2) bildet, und
**dadurch gekennzeichnet, dass** die Filterpatronenanordnung (1) ferner umfasst:
- eine Filterdichtung (8), um jeweilige Filterkappen (4) an jedem der vorgeformten Filtergehäuse (5) abzudichten, wobei jedes der vorgeformten Filtergehäuse (5) einen Dichtungssitz (5b) umfasst, in dem die Filterdichtung (8) mit dem einteiligen Basisteil (7) verbunden ist, um jede Filterkappe (4) an den vorgeformten Filtergehäusen (5) abzudichten,
- wobei der Flüssigkeitspfad (6) jeder Filterpatrone (2) einen Filtereinlass (6a), der an der Filterkappe (4) angeordnet ist, und einen Filterauslass (6b), der an dem jeweiligen vorgeformten Filtergehäuse (5) angeordnet ist, umfasst, wobei der Filtereinlass (6a) konfiguriert ist, Flüssigkeit von einer Spritzeninjektionsvorrichtung einer automatisierten Auflösungsprobenstation aufzunehmen, und
- wobei die Filterpatronenanordnung (1) konfiguriert ist, in ein Filtermodul der automatisierten Auflösungsprobenstation eingesetzt werden zu können.

2. Filterpatronenanordnung (1) nach Anspruch 1, wobei jede Filterkappe (4) eine umlaufende Lasche (9) enthält, die konfiguriert und derart angeordnet ist, dass die umlaufende Lasche (9) dann, wenn sie mit ihrem entsprechenden vorgeformten Filtergehäuse (5) zusammengebaut wird, auf einem Abschnitt des Dichtungssitzes (5b) liegt und mit der Filterdichtung (8) verbunden ist.

3. Filterpatronenanordnung (1) nach einem der Ansprüche 1 bis 2, wobei jedes der vorgeformten Filtergehäuse (5) ferner einen Filtersitz (5c) für das Filtermedium (3) umfasst und wobei ein umlaufender vorspringender Abschnitt (10) jeder Filterkappe (4) das Filtermedium (3) zwischen dem Filtersitz (5c) jedes vorgeformten Filtergehäuses (5) und dem umlaufenden vorspringenden Abschnitt (10) jeder Filterkappe (4) einklemmt.

4. Filterpatronenanordnung (1) nach einem der Ansprüche 1 bis 3, wobei das einteilige Basisteil (7) mehrere miteinander verbundene Sitzabschnitte (7a) umfasst, in denen die Filterdichtung (8) auch mit dem einteiligen Basisteil (7) verbunden ist.

5. Filterpatronenanordnung (1) nach einem der Ansprüche 1 bis 4, wobei die Filterdichtung eine einteilige Filterdichtung (8) zum Abdichten der Filterkappen (4) der mehreren Filterpatronen (2) ist.

6. Filterpatronenanordnung (1) nach einem der Ansprüche 1 bis 5, wobei die Filterdichtung (8) durch Umspritzen mit dem einteiligen Basisteil (7) verbunden ist.

7. Filterpatronenanordnung (1) nach einem der Ansprüche 1 bis 6, wobei die Filterdichtung (8) eine Farbe aufweist, bevorzugt eine Farbe, die sich von der Farbe jeder Filterkappe (4) und der Farbe des einteiligen Basisteils (7) unterscheidet.

8. Filterpatronenanordnung (1) nach einem der Ansprüche 1 bis 7, wobei das einteilige Basisteil (7) mindestens eine verstärkte Rippe (11) umfasst, die zwischen den vorgeformten Filtergehäusen (5) angeordnet ist.

9. Filterpatronenanordnung (1) nach einem der Ansprüche 1 bis 8, wobei jedes vorgeformte Filtergehäuse (5) eine gefurchte Oberfläche (5) umfasst, die einen gefurchten Strömungsweg zum Führen der gefilterten Probe zu dem Filterauslass (6b) definiert.

10. Filterpatronenanordnung (1) nach einem der Ansprüche 1 bis 9, wobei das Filtermedium eine Filtermembran (3) umfasst, die konfiguriert und auf jedem der vorgeformten Filtergehäuse (5) angeordnet ist, um partikelförmige Verunreinigungen und/oder Bakterien aus zu filternden Flüssigkeitsproben zu entfernen.

11. Verfahren zum Erhalten einer Filterpatronenanordnung (1) für automatisierte Probenpräparation bei Auflösungsprüfung nach einem der Ansprüche 1 bis 10, wobei die Anordnung mehrere Filterpatronen (2) enthält und jede der Filterpatronen (2) einen Flüssigkeitspfad (6) für eine zu filternde Flüssigkeitsprobe durch ein Filtermedium (3) enthält, wobei das Verfahren die Schritte umfasst:
a. Bereitstellen eines einteiligen Basisteils (7), das mehrere vorgeformte Filtergehäuse (5) definiert, wobei jedes vorgeformte Filtergehäuse (5) einen integralen Teil einer Filterpatrone (2) der mehreren Filterpatronen (2) bildet,
b. Anordnen einzelner Filtermedien (3) auf jedem der mehreren vorgeformten Filtergehäuse (5),
c. Bedecken jedes der mehreren vorgeformten Filtergehäuse (5) mit einer jeweiligen Filterkappe (4), und
d. Abdichten der jeweiligen Filterkappen (4) an jedem der vorgeformten Filtergehäuse (5) durch Verbinden einer Filterdichtung (8) mit jeweiligen Dichtungssitzen (5b) der vorgeformten Filtergehäuse (5).

12. Verfahren nach Anspruch 11, wobei Schritt d) Verbinden der Filterdichtung (8) mit den jeweiligen Dichtungssitzen (5b) der vorgeformten Filtergehäuse (5) durch Umspritzen umfasst.

13. Verfahren nach einem der Ansprüche 11 oder 12, das den Schritt von Vorbelasten des einteiligen Basisteils (7) vor Verbinden der Filterdichtung (8) mit den jeweiligen Dichtungssitzen (5b) der vorgeformten Filtergehäuse (5) umfasst.

## Revendications

1. Ensemble de cartouches filtrantes (1) pour une préparation d'échantillons automatisée dans un test de dissolution comprenant une pluralité de cartouches filtrantes (2), chacune desdites cartouches filtrantes (2) incluant un trajet de liquide (6) pour un échantillon de liquide à filtrer à travers un milieu filtrant (3), dans lequel l'ensemble de cartouches filtrantes (1) comprend
□ une partie de base monobloc (7) définissant une pluralité de boîtiers de filtre préformés (5), dans lequel chacun desdits boîtiers de filtre préformés (5) fait partie intégrante de l'une desdites cartouches filtrantes (2) de ladite pluralité de cartouches filtrantes (2), et
**caractérisé en ce que** l'ensemble de cartouches filtrantes (1) comprend en outre
□ un joint d'étanchéité de filtre (8) pour sceller des bouchons de filtre respectifs (4) à chacun desdits boîtiers de filtre préformés (5), dans lequel chacun desdits boîtiers de filtre préformés (5) comprend un siège d'étanchéité (5b) où le joint d'étanchéité de filtre (8) est lié à la partie de base monobloc (7) pour sceller chaque bouchon de filtre (4) à chacun desdits boîtiers de filtre préformés (5),
□ dans lequel le trajet de liquide (6) de chaque cartouche filtrante (2) comprend une entrée de filtre (6a) agencée sur le bouchon de filtre (4), et une sortie de filtre (6b) agencée sur le boîtier de filtre préformé respectif (5), dans lequel ladite entrée de filtre (6a) est configurée pour recevoir du liquide à partir d'un dispositif d'injection à seringue d'une station d'échantillon de dissolution automatisée, et
□ dans lequel ledit ensemble de cartouches filtrantes (1) est configuré pour pouvoir être inséré dans un module de filtre de la station d'échantillon de dissolution automatisée.

2. Ensemble de cartouches filtrantes (1) selon la revendication 1, dans lequel chaque bouchon de filtre (4) inclut une languette périphérique (9) configurée et agencée de telle sorte que, lorsqu'elle est assemblée à son boîtier de filtre préformé correspondant (5), la languette périphérique (9) repose sur une partie du siège d'étanchéité (5b) et est liée au joint d'étanchéité de filtre (8).

3. Ensemble de cartouches filtrantes (1) selon l'une quelconque des revendications 1 à 2, dans lequel chacun desdits boîtiers de filtre préformés (5) comprend en outre un siège de filtre (5c) pour le milieu filtrant (3), et dans lequel une partie saillante périphérique (10) de chaque bouchon de filtre (4) serre ledit milieu filtrant (3) entre le siège de filtre (5c) de chaque boîtier de filtre préformé (5) et la partie saillante périphérique (10) de chaque bouchon de filtre (4).

4. Ensemble de cartouches filtrantes (1) selon l'une quelconque des revendications 1 à 3, dans lequel ladite partie de base monobloc (7) comprend une pluralité de parties de siège d'interconnexion (7a) où le joint d'étanchéité de filtre (8) est également lié à la partie de base monobloc (7).

5. Ensemble de cartouches filtrantes (1) selon l'une quelconque des revendications 1 à 4, dans lequel le joint d'étanchéité de filtre est un joint d'étanchéité de filtre monobloc (8) pour sceller les bouchons de filtre (4) de la pluralité de cartouches filtrantes (2).

6. Ensemble de cartouches filtrantes (1) selon l'une quelconque des revendications 1 à 5, dans lequel le joint d'étanchéité de filtre (8) est lié par surmoulage à la partie de base monobloc (7).

7. Ensemble de cartouches filtrantes (1) selon l'une quelconque des revendications 1 à 6, dans lequel le joint d'étanchéité de filtre (8) présente une couleur, de préférence, une couleur différente de la couleur de chaque bouchon de filtre (4) et de la couleur de la partie de base monobloc (7).

8. Ensemble de cartouches filtrantes (1) selon l'une quelconque des revendications 1 à 7, dans lequel la partie de base monobloc (7) comprend au moins une nervure renforcée (11) agencée entre lesdits boîtiers de filtre préformés (5).

9. Ensemble de cartouches filtrantes (1) selon l'une quelconque des revendications 1 à 8, dans lequel chaque boîtier de filtre préformé (5) comprend une surface canalisée (5) définissant un trajet d'écoulement canalisé pour guider l'échantillon filtré vers la sortie de filtre (6b).

10. Ensemble de cartouches filtrantes (1) selon l'une quelconque des revendications 1 à 9, dans lequel le milieu filtrant comprend une membrane filtrante (3) configurée et agencée sur chacun desdits boîtiers de filtre préformés (5) pour l'élimination d'impuretés particulaires et/ou de bactéries à partir d'échantillons liquides à filtrer.

11. Procédé d'obtention d'un ensemble de cartouches filtrantes (1) pour la préparation automatisée d'échantillons dans un test de dissolution selon l'une quelconque des revendications 1 à 10, dans lequel ledit ensemble inclut une pluralité de cartouches filtrantes (2) et chacune desdites cartouches filtrantes (2) inclut un trajet de liquide (6) pour un échantillon liquide à filtrer à travers un milieu filtrant (3), comprenant les étapes consistant à :
a. fournir une partie de base monobloc (7) définissant une pluralité de boîtiers de filtre préformés (5), dans lequel chaque boîtier de filtre préformé (5) fait partie intégrante d'une cartouche filtrante (2) de ladite pluralité de cartouches filtrantes (2),
b. agencer des milieux filtrants individuels (3) sur chacun de ladite pluralité de boîtiers de filtre préformés (5),
c. recouvrir chacun de ladite pluralité de boîtiers de filtre préformés (5) avec un bouchon de filtre respectif (4), et
d. sceller les bouchons de filtre respectifs (4) à chacun desdits boîtiers de filtre préformés (5) en reliant un joint d'étanchéité de filtre (8) aux sièges d'étanchéité respectifs (5b) desdits boîtiers de filtre préformés (5).

12. Procédé selon la revendication 11, dans lequel l'étape d) comprend une liaison par surmoulage du joint d'étanchéité de filtre (8) aux sièges d'étanchéité respectifs (5b) desdits boîtiers de filtre préformés (5).

13. Procédé selon l'une quelconque des revendications 11 ou 12, comprenant l'étape consistant à précontraindre la partie de base monobloc (7) avant de lier le joint d'étanchéité de filtre (8) aux sièges d'étanchéité respectifs (5b) desdits boîtiers de filtre préformés (5).
